# EUROPEAN PATENT APPLICATION

(11) **EP 1 291 435 A2**
(43) Date of publication of application: **12.03.2003**
(21) Application number: 02019346.2
(22) Date of filing: 29.08.2002
(51) Int. Cl.: C12P 7/40, C12P 13/00, C12P 13/02

(54) **A micro-organism possessing enantioselective and regioselective nitrile hydratase/amidase activities**

(30) Priority: 30.08.2001 IT MI20011826
(71) Applicant: ISTITUTO BIOCHIMICO ITALIANO GIOVANNI LORENZINI S.p.A., 20134 Milan (IT)
(72) Inventor: Salvo, Giuseppe, 98056 Mazzarra'Sant'Andrea (Messina) (IT); Brandt, Alberto, 00183 Roma (IT); Cecchetelli, Loredana, 00045 Genzano (Rome) (IT)
(74) Representative: Gervasi, Gemma, Dr.

(57) **Abstract**

The present invention is concerned with new micro organisms, preferably mutagenised, belonging to the genus *Agrobacterium radiobacter* able to convert nitriles and/or amides into their respective acids, in addition to conversion processes utilising said microorganisms.

## Description

### FIELD OF THE INVENTION.

The present invention is concerned with new micro-organisms, preferably mutagenised, belonging to the genus *Agrobacterium radiobacter* able to hydrate and/or hydrolyse nitriles or amides, in addition to conversion processs utilising said micro-organisms.

### PRIOR ART.

The enzymatic conversion of nitriles to acids is advantageously obtained in the known art with enzymes derived from micro-organisms, in as much as the corresponding traditional "chemical" transformation requires drastic conditions (6M HCl or 2M NaOH with reflux) and is not easily performed with the chemo-, regio- and stereoselectivity as well as the stereospecificity and in particular with the enantioselectivity often required, e.g. in the synthesis of some speciality chemicals and above all pharmaceuticals.

Amongst the more common applications of enzymes derived from micro-organisms capable of processing nitriles, are noted the production of acrylamide from acrylonitrile (Kobayashi, M. et al., Eur. J. Biochem. 217: 327-336 1993), environmental decontamination processes (Kobayashi, M. et al., J. Bacteriology 172: 4807-4815, 1990) and the production of textile fibres (Tauber, M. M. et al., Appl. and Environ. Microbiol. 56: 3125-3129, 2000). For example, the bacterium *Rhodococcus rhodochrous* K22 shows an activity both towards saturated aliphatic nitriles (e.g. valeronitrile or glutaronitrile) and for unsaturated nitriles (e.g. crotonitrile) (Kobayashi, M. et al., FEMS Miocrobiol. Lett. 77, 121-124 1991 and FEMS Miocrobiol. Lett. 120, 217-224 1994). It lacks however activity towards aromatic nitriles.

Generally the micro-organisms display either nitrilase activity (that is the hydrolysis of nitriles directly to give the respective acid), isolated for example from bacteria of the genus *Alcaligenes Faecalis, Rhodococcus rhodochrous* K22, *Acinetobacter* sp. strain AK226, *Acidovorax facilis* 72 W) or nitrile hydratase/amidase activities (that is, in a first step, hydration of the nitrile to give the respective amide which is then, in a second step, in turn hydrolysed giving the corresponding acid) isolated for example from bacteria of the genus *Rhodococcus rhodochrous* J1, *Rhodococcus* sp. 361, *Rhodococcus* sp. C3II, *Rhodococcus erythropolis* MP50, *Agrobacterium tumefaciens, Camomonas testosteroni, Candida famata, Brevibacterium* sp. R312. More rarely both activities have been isolated from the same micro-organism, as for example in *Rhodococcus butanica,* ATCC cat. 21197 (US 3,751,337) or in *Arthrobacter* sp. strain J1.

For example, in the pharmaceutical industry, this type of enzyme activity, in cases in which it possesses a marked enantioselectivity towards specific chiral substrates constituting precursors of active ingredients, is exploitable in the production of optically active α-aryl-propionic acids ("profens") which constitute a class of potent, non-steroid anti-inflammatory agents and of which the respective S-enantiomers have shown themselves pharmacologically preferable with respect to the R-enantiomers and therefore of which preparations of the "enantiopure" S-enantiomer are preferred to the racemic form initially commercialised. Consequently, different groups of researchers have examined the possibility of utilising the enzyme pair nitrile hydratase/amidase of strains of *Rhodococcus erythropolis* MP50 and C3II for the production of S-naproxen and S-ketoprofen.

An enzyme with nitrilase activity towards R,S-ibuprofen nitrile for the enantioselective production of S-(+)-ibuprofen is obtainable from *Acinetobacter* sp. AK226 as described by Yamamoto et al., Agric. Biol. Chem. 55 86), 1459-1466, 1991. The enzyme, which acts on a vast quantity of substrates (symmetrical and chiral) such as aromatic nitriles (e.g. benzonitrile), arylalkyl nitriles (e.g. 2-phenylpropionitrile), aliphatic nitriles (e.g. acrylonitrile) and heterocyclic nitriles (e.g. 2-furonitrile), in addition to dinitrile compounds (for example malononitrile), has shown itself to then be highly enantioselective for (S)-(-)Ibu-CN, which is hydrolysed more quickly than its corresponding mirror form R-(+)-Ibu-CN, thus obtaining S(+)-ibuprofen in notable enantiomeric excess. One is therefore dealing with a new nitrilase capable of choosing between the two types of substrate (enantiomers).

Nevertheless, in enzymatic systems comprising both nitrile hydratase and amidase activities, the activity (and also the enantioselectivity) in the two respective phases can be markedly different. In particular, one finds predominantly that the nitrile hydratase enzymes of microbial origin preferentially give the R-enantiomer, whilst the amidases preferentially give the S-enantiomer. (Cohen, M. A. et al., Tetrahedron: Asymmetry 3: 1543-1546 1992; Kakeya, H. et al., Tetrahedron Letters 32: 1343-1346, 1991; Layh, N. et al., J. Biotechnology 33: 175-182, 1994; Hirrlinger, B. et al., J. Bacteriology 178: 3501-3507, 1996; Effenberger, F. et al., J. Biotechnology 60: 165-174, 1998). In this last work, Effenberger and co-authors also examine further the regioselectivity of the biocatalytic system and demonstrate that, on the basis of activity and preference for different substrates, the two strains C3II and MP50 can be complimentary in the regioselective conversion of nitriles to amides and of amides to acids: strain C3II hydrolyses the dinitrile to give the corresponding mononitrile/monoamide, whilst the strain MP50 allows the production of the monoacids, that is of the mononitrile/monoacid and of the monoamide/monoacid. They use aliphatic, unsaturated and aromatic dinitriles as substrate. Other strains which utilise aliphatic dinitriles in which the cyano groups are found, in the α and ω positions, respectively, as substrates are *Acidovorax facilis* and *Camomonas testosteroni.* Of the two, only the first has a nitrilase which directly converts the α,ω-dinitriles to ω-cyanocarboxylic acid. The second, however, has a nitrile hydratase/amidase system which gives ω-cyanocarboxylic acid through the corresponding amide (Gavagan, J. and et al., J. Org. Chem. **63:** 4792-4801, 1998).

Bauer et al. (*Appl. Microbiol. Biotechnol.* **42:** *1-7* (1994) and *Appl. Microbiol. Biotechnol.* **49:** 89-95 (1998)) describe a nitrile hydratase/amidase system of *Agrobacterium tumefaciens,* strain d3. Whilst in the first article the authors observe both whole cells, and cell extracts and demonstrate the preference of Agrobacterium tumefaciens to process the (S)-enantiomer of a given racemic substrate (nitrile or amide) in both phases of the enzymatic transformation (with the important exception of Ibuprofen nitrile which gives the corresponding (R)-Ibuprofen, that is the least favoured enantiomer from a pharmacological view point), the same authors then demonstrate in the second article that the nitrile hydratase purified enzyme is highly stereospecific with respect to many phenylpropionitrile compounds because it preferentially converts the S-enantiomer to selectively give the corresponding S-phenylpropionamide (for example, it reaches e.e.>90%, at 30% yield, for S-ketoprofen amide). Still in the second article is further described the influence of temperature on the nitrile hydratase reaction with whole cells in the presence of an inhibitor of amidase activity. Previously, in the first article, Bauer et al. Have investigated the nitrogen source utilisable for the growth of the micro-organism and for the induction of its enzyme activity, establishing that *Agrobacterium tumefaciens* d3 described by Bauer et al. can be grown by the use of various organic nitriles e.g. 2-phenylproprionitrile as a source of nitrogen and also as inducers of enzyme activity, but that the latter is totally suppressed in the case where ammonium is present in the culture medium.

In the patent US 5,395,758 from Sumimoto Chemical Company Ltd is described instead a micro-organism of the genus *Agrobacterium radiobacter,* strain SC-C15-1 (deposited according to the treaty of Budapest on 23 April 1992 with accession number FERM BP-3843), able to convert aliphatic and aromatic nitriles into their corresponding amides. US 5,395,758 does not however contain any indication on the enantioselectivity of Agrobacterium radiobacter strain SC-C15-1; in particular there is no mention of the optical yield possibly obtainable starting from racemic nitriles. Furthermore, for example according to example 4 of US 5,395,758 a "whole cell catalyst" constituted of *Agrobacter radiobacter* strain SC-C15-1, and characterised by the fact of converting nitrile substrates quantitatively into amides, without the subsequent formation and therefore the possibility of the isolation of acids. It is therefore to be noted that *Agrobacterium radiobacter* strain SC-C15-1 lacks amidase activity.

Also in Gabriel et al. (J. Chromatology B, 1996, 681:191-195) is described a micro-organism of the genus *Agrobacterium radiobacter* which degrades aromatic nitriles, used as pesticides. However also *Agrobacterium radiobacter* 8/4, as identified and described by the authors of this article, results as being lacking in amidase activity, in as much as the nitrile pesticides offered as substrates are always metabolised to give the respective amides. Furthermore, no indication regarding the possible enantioselectivity of *Agrobacterium radiobacter* 8/4 with respect to certain substrates is given.

One can therefore conclude that all the strains of the genus *Agrobacterium* (species *radiobacter* or *tumefaciens)* as above are wild type strains showing predetermined enzymatic characteristics. For example, none of the micro-organisms of the species

*Agrobacterium radiobacter* in the known art demonstrated an amidase activity.

One object of the present invention is therefore the making available of a new strain of *Agrobacterium radiobacter* which shows an amidase activity.

A further particularly preferred object is the making available of a new strain of the genus *Agrobacterium radiobacter* mutagenised with optimised enzymatic activity, that is both, preferably increased and demonstrating a distinct regioselectivity, stereospecificity and/or enantioselectivity. It is particularly preferred that this optimised enzymatic activity can be induced with low-cost inducers.

A further object of the present invention is the making available of a conversion process, preferably enantioselectively, of nitriles into their respective acids, possibly comprising the stage a) of conversion of nitriles to amides and that of b) of conversion of amides to acids. A further object of the present invention is the making available of a process for the conversion, preferably enantioselectively, of amides into their respective acids.

A further object of the present invention is the making available of a process for the conversion, preferably enantioselectively, of nitriles into their respective amides.

A further object of the present invention is the making available of a process for the regioselective hydratation and/or hydrolysis of dinitriles.

### SUMMARY OF THE INVENTION.

The said objects and further objects, that will be better described below, are achieved through the present invention which is concerned with a new micro-organism belonging to the genus *Agrobacterium radiobacter,* capable of converting nitriles and/or amides into the respective acids. According to a preferred embodiment of the present invention, a mutagenised micro-organism belonging to the genus *Agrobacterium radiobacter,* catalase-positive and oxidase-negative, with optimised, i.e. increased enzymatic activity and showing a distinct regioselectivity, stereoselectivity, and/or enantioselectivity is provided. According to a particularly preferred embodiment of the present invention *Agrobacterium radiobacter* 30"60 (NCIMB 41108) is provided. Furthermore, the present invention provides a series of processes for the hydratation and/or hydrolysis of nitriles or amides which utilise the micro-organisms provided by the present invention.

### DESCRIPTION OF THE DRAWING.

To best illustrate the present invention, the following Figures are attached, in which:
Figure 1 represents the layout of the selection process of the micro-organisms in general, and
Figure 2 illustrates the genealogy of *Agrobacterium radiobacter* 30"60 (NCIMB 41108) following mutagenesis.

### DETAILED DESCRIPTION OF THE INVENTION.

An object of the present invention is therefore a new micro-organism, preferably mutagenised, belonging to the genus *Agrobacterium radiobacter,* capable of converting nitriles and/or amides into their respective acids. A further object of the present invention is a series of processes for the hydratation and/or hydrolysis of nitriles or amides which utilise new micro-organisms of the genus *Agrobacterium radiobacter.*

The conversions are carried out enzymatically and on that account the micro-organism is defined for the purpose of the present invention, as a biocatalyst.

The new micro-organism according to the present invention belongs to the family of the Rhizobiacee genus II, *Agrobacterium radiobacter,* and displays the following morphological characteristics: has a rod-like shape of approx. 0.8 µm by 1.5-3 µm.

Appears to have motility under light microscopy, does not form spores and is Gram-negative following staining carried out using methods known in the state of the art. It grows at a pH comprised of between 6-9 and at a temperature comprised of between 5 and 45°C in medium containing yeast extract, peptone, dextrose, NaCl. In particular, the micro-organism according to the present invention uses a vast plurality of carbohydrates, organic acid salts and/or aminoacids as carbon sources, whilst cellulose, glucose agar, D-galactose and other carbohydrates are not used. Amongst the nitrogen sources utilisable should be named ammonium salts, but not nitrates which are not utilised. A preferred realisation of the invention is constituted by a mutagenised micro-organism denominated *Agrobacterium radiobacter 30"60* better described below and deposited under the treaty of Budapest on 3rd May 2001 with the NCIMB (National Collection of Industrial and Marine Bacteria Ltd, Aberdeen, Scotland, Great Britain), with accession number 41108. It is to be pointed out that *Agrobacterium radiobacter* 30"*60* (NCIMB 41108) constitutes a new strain.

Consequently, in addition to this new strain, the present invention makes also available any mutant derivable in turn from *Agrobacterium radiobacter* 30"*60* (NCIMB 41108), for example any strain obtained by cell fusion or any recombinant strain derived from *Agrobacterium radiobacter* 30"*60* (NCIMB 41108).

*Agrobacterium radiobacter 30*"60 (NCIMB 41108) which is further characterised and described in the experimental section of the present patent application, like the wild-type strain from which it was obtained, is an obligatory aerobe and, as said above, does not reduce nitrates. Starting from the freeze dried strain, after 48 hours of growth on solid medium forms rough, round colonies, about 1.2 mm in diameter and of an ivory/white colour. As described above, *Agrobacterium radiobacter 30*"60 (NCIMB 41108) can utilise ammonium salts as sources of nitrogen. It is therefore important to underline that the use of ammonium salts does not then bring about inhibition of its enzymatic activity towards nitrile substrates, as it has been described however e.g. for strain d3 of *Agrobacterium tumefaciens* (Bauer et al., "Enantioselective hydrolysis of racemic 2-phenylproprionitrile and other (*R,S*)-2-arylproprionitriles by a new bacterial isolate, *Agrobacterium tumefaciens* strain d3. *Appl. Microbiol. Biotechnol.* **42**: 1-7, 1994).

According to the-classification API 20 NE, *Agrobacterium radiobacter* 30"*60* (NCIMB 41108) results furthermore as being positive for the enzyme marker catalase. Contrary to the strain of *Agrobacterium radiobacter* SC-C15-1 described in US 5,395,758, *Agrobacterium radiobacter* 30"*60 (NCIMB 41108)* is not capable of reducing nitrates, does not use citrate as a sole carbon source, results as being negative to the oxidase test and does not produce acid from glucose. The minimum agar medium, on which has been selected the strain *Agrobacterium radiobacter* 30"*60* (NCIMB 41108) and on which it shows optimal growth, comprises at least the following constituents: KH₂PO₄ (1 g 1⁻¹), Na₂ HPO₄ (3 g l⁻¹), MgSO₄ (0.5 g l⁻¹), NaCl (1 g l⁻¹), CaCl₂.6H₂O (0.1 g l⁻¹), FeSO₄ (0.4 g l⁻¹), CoCl₂ (0.1 g l⁻¹), MnSO₄ (0.01 g l⁻¹), lactamide (2,5 g l⁻¹), succinonitrile (2,5 g l⁻¹), extra pure Agar (20 g l⁻¹). Some authors (Brammar et al., J. Gen. Microbiol. **47**: 87-102, 1967; Betz and Clarke, J. Gen. Microbiol. 73: 161-174, 1972, Smyth and Clarke, J.Gen. Microbiol. **90**: 81-90, 1975) describe with reference to *Pseudomonas aeruginosa* that amidase synthesis is repressed by the use of succinate and by the combination of succinate as a carbon source with an amide as a nitrogen source. Furthermore the same authors describe, that this type of medium constitutes an efficient selective medium for the isolation of amidase regulatory mutants, of mutants with altered enzyme and of mutants resistant to catabolite repression. The fermentation medium later developed for *Agrobacterium radiobacter 30"60* (NCIMB 41108) is comprised of: MgSO₄ (0.5 g l⁻¹), CaCl₂.6H₂O (0.1 g l⁻¹), FeSO₄ (0.4 g l⁻¹), CoCl₂ (0.1 g l⁻¹), MnSO₄ (0.01 g l⁻¹), (NH₄)₂ SO₄ (5 g l⁻¹), yeast extract(10 g l⁻¹), glucose (5 g l⁻¹). The essential components for enzymatic activity results as being the ions of Ca, Co, Fe, Mg and Mn; extremely important are the ions of Co in their various oxidation states, in particular as Co²⁺.

In this medium the strain *Agrobacterium radiobacter* 30"60 (NCIMB 41108) reaches a maximum density of 18.5-19 mg of cells /ml (9 x 10⁹ - 1.1 x 10¹⁰ cells/ml), corresponding to an optical density (sample diluted 1:10) measured at 600 nm of 0.9 absorbance units. The bacterium *A. radiobacter 30"60* (NCIMB 41108) shows a basal activity for the enantioselective conversion of nitriles and amides into their respective acids, which is induced notably through addition to the culture medium of nitriles and/or amides, both either aliphatic, arylalkyl or aromatic in nature. The strain is therefore definable as semi-constitutive.

Preferred but not exclusive examples of nitrile inducers of enzymatic activity are "simple" nitriles or dinitriles or non-chiral nitriles or dinitriles, preferably aliphatic, branched or linear comprised of 2-12 carbon atoms. Amongst these particularly preferred are the non-chiral aliphatic nitriles such as for example valeronitrile, isovaleronitrile, butyronitrile, isobutyronitrile, ε-caprolactam, or non-chiral aliphatic dinitriles. Whilst it is also possible to induce the bacterium *Agrobacterium radiobacter 30"60* (NCIMB 41108) with other nitriles, e.g. arylalkyl or aromatic or with chiral nitriles (e.g. with (R,S)-2-phenylproprionitrile, an arylalkyl nitrile, used sometimes in the prior art), the nitrile hydratase/amidase activity thus induced is acceptable but however inferior to that obtained with simple aliphatic nitriles and dinitriles which remain preferred. Amongst the amides, which can also be aliphatic, arylalkyl or aromatic, and which comprise also the lactams, are used preferably the following molecules as inducers: ε-caprolactams, isobutyramide, benzamide, valeramide, butyramide and lactamide and can therefore be defined as "simple" amides within the scope of the present invention. Particularly preferred are valeronitrile and butyronitrile, utilised, as all of the inducers, at a concentration which is non-toxic for the micro-organism, for example at a final concentration comprised of between 0.5-5 g/litre. The inducer, or the inducers, are added to the fermentation medium alone or in combination, in a sterile manner.

The possibility of inducing the nitrile hydratase/amidase enzymatic activity to high levels with low-cost molecules, preferably with "simple" nitriles and/or amides as above, represents an advantage of the biocatalysts according to the present invention, with respect to that known in the art: in fact very frequently, good levels of induction were obtained exclusively with the same reaction substrate intended for catalysis, or with "model substances", often relatively expensive, simulating as much as possible the real substrate of the synthesis reaction, as it is for example in the case of (R,S)-2-phenylproprionitrile frequently used in the experimentation described in the known art to induce the capacity to then process certain chiral nitriles and amides convertible into the respective profens of pharmacological interest.

A further object of the invention is therefore constituted by a series of processes for the hydratation and/or hydrolysis of nitriles or amides which use the new micro-organisms *Agrobacterium radiobacter* according to the invention.

Amongst these conversion processes of the present invention, the following should be named: the conversion of nitriles into their respective acids, the-conversion of nitriles into their respective amides, the conversion of amides into their respective acids and the regioselective hydratation or hydrolysis of dinitriles.

Particularly preferred are therefore the conversion processes which utilise the strain *Agrobacterium radiobacter* 30"60, deposited with accession number 41108 on 3^{rd} of May 2001 with the NCIMB.

According to a preferred but not exclusive embodiment of the present invention, in the conversion reactions, the micro-organisms of the strain *Agrobacterium radiobacter* 30"60 (NCIMB 41108) are used as a bacterial "pellet" (whole cell catalyst) obtained after fermentation in growth medium containing the inducer or the inducers as described below. The seed-medium (inoculation medium) is inoculated with a single colony of the strain *Agrobacterium radiobacter* 30"60 (NCIMB 41108) taken from a plate culture, or sloping test tube, grown for 18-36 hours thermostatically at a temperature of 30° C. The micro-organism utilised for the inoculum, derives from bacterial stock lyophilised or frozen in the presence of glycerol at a temperature of-80° C. The liquid culture utilisable as an inoculum, is grown in a shaker (conical flask subjected to agitation) as an aerated culture at 180 rpm and at a temperature of 30° C, for a maximum of 24 hours, best, up to 22 hours where the strain *Agrobacterium radiobacter* 30"60 (NCIMB 41108) reaches a maximum density of 13.1 mg of cells/ml (5.8 x 10 ⁹ cells/ml) corresponding to an optical density (sample diluted 1:10) measured at 600 nm of 0.588 absorbance units. The "fermentation medium", is then inoculated with a volume of bacterial culture equal to 1%-50%, preferably at 10 % (v/v) of the total and the process started as an aerated culture in a shaker at 300 rpm, at a temperature of 30° C and for a time varying from 24 to 96 hours, best if for 48 hours. The total volume of medium, utilised for a 500 ml flask, is in general 100 ml. The inducer or the mixture of inducers, is added sterilely as a single solution at the start of the process.

The bacterial *pellet,* obtained by centrifugation of the induced bacterial culture, and washed appropriately through washes in isotonic saline or water, constituting the whole cell biocatalyst and is in the form of a wet bacterial paste. It is stored at a temperature below that of 10°C, preferably comprised of between 3°C and 6°C, still more preferably 4°C. The whole cell catalyst constitutes in fact a preferred but not exclusive embodiment of the present invention. However, comprised within the scope of the present invention and as such embraced by the definitionof biocatalysts according to the invention, are micro-organism preparations constituted by lysed bacteria, also only partially, for example through enzymatic, chemical or physical treatment, or crude bacterial extracts, for example obtained by complete cellular lysis such as these obtainable through treatment of a bacterial suspension with a French-press, or still homogenates, for example obtained with Potter or sonicator homogenisers, or semi-purified preparations of the bacterial extract such as these obtained by centrifugation of a crude bacterial lysate, in the presence of appropriate flocculants.

The conversion reactions catalysed by the micro-organisms according to the present invention, take place at a pH comprised of between 5 and 9.5, preferably comprised of between 7 and 8, in buffered solutions, such as for example acetate buffer, Tris-HCl buffer and phosphate buffer, phosphate buffer at pH 7.7 being particularly preferred. The reaction temperature is comprised of between 5 and 45°C, more preferably between 28 and 42°C and still more preferably between 38 and 42°C. The substrate can be, if insoluble in buffer, solubilised in organic solvents, such as alcohol, e.g. ethanol, methanol, isopropanol or tert-butanol, or in organic solvents such as dioxane, N,N-dimethylformamide, dimethylsulphoxide, etc. Particularly preferred is methanol, present in the reaction mixture, as with all the organic solvents above, at a final concentration compatible with the metabolic systems of the micro-organism, best if it is not greater than 10% (v/v). To the reaction mixture can also be added detergents, preferably anionic, such as Tween®-20, Tween®-80 (or other Tweens® listed for example in the "SIGMA" catalogue), or various Tritons® (for example Triton® X-100 or X-114, X-405, N-101, CG 110, XL-80N, WR-1339 or others still, as listed for example in the "SIGMA" catalogue) in final concentrations, always "non toxic" for the reaction system, preferably comprised of between 0.5-5%.

The biocatalyst is added to the reaction solution in weight ratios comprised of between 10 and 0.5 (bacteria/substrate). The weight of the biocatalyst is expressed as wet weight of bacterial paste. In the case of bacterial extracts or of liquid homogenates, the ratio is adapted in consideration of the wet weight of the bacteria initially processed.

With reference to the enzymatic activity of the biocatalysts discussed within the context of the present invention, it should be noted that these results are modifiable by experts in the field through the variation of the growth conditions-of the biocatalyst. For example, the activities can be optimised, if required, through appropriate variations to the culture medium, of the fermentation times and/or to the physico-chemical and mechanical parameters (for example the aeration) of growth.

Amongst the convertible substrates using the biocatalyst of the present invention are to be named nitriles of formula R-CN, in which R constitutes an organic residue. Aliphatic nitriles in which R is an aliphatic group, arylalkyl nitriles in which R is an arylalkyl group and aromatic nitriles in which R is an aromatic group are preferred.

Amongst the aliphatic nitriles, the preferred aliphatic nitriles according to the present invention are these in which R is a linear, branched or cyclic aliphatic group and is comprised of 1-20 carbon atoms and if necessary 1-2 heteroatoms selected from O, S, N. R can be saturated or contain 1-4 double bonds and/or 1-3 triple bonds. Furthermore, R can be substituted by 1-5 of the following groups: F, Cl, Br, I, OH, SH, NH₂, CHO, COOH, cyclopropyl, cyclobutyl, cyclopentyl, cyclohexyl, cycloheptyl. In the case of the presence of cycloaliphatic groups, these may contain 1-2 heteroatoms selected from O, S, N, may contain 1-2 double bonds and may be substituted in turn by 1-3 of the following groups: F, Cl, Br, I, OH, SH, NH₂, CHO, COOH or C₁-C₆-alkyl (linear or branched, comprised of 1-2 double bonds or a triple bond), in its turn substituted by 1-3 of the following groups: F, Cl, Br, I, OH, SH, NH₂, CHO, COOH.

Aliphatic nitriles particularly preferred according to the present invention are 2-piperidylnitrile, 2-methylbutyrronitrile, 2-methylpentanonitrile, 3-methyl-pentanonitrile and 2-methylhexanonitrile.

Amongst the arylalkyl nitriles are preferred for example some α-methyl-arylacetonitriles (or "arylpropionitriles"), especially nitriles which (-through the formal replacement of CN with COOH-) are the precursors of the class of pharmaceutical compounds denominated profens. A series of arylalkyl nitriles particularly preferred according to the present invention is constituted by aliphatic nitriles as above substituted in their turn by 1-4 aryl groups ("aromatics") or heteroaryl ("heteroaromatics") comprised of 4-14 carbon atoms and, optionally, 1-3 heteroatoms selected from O, S, N. Amongst the aryl groups to be mentioned for example are the phenyl and naphthyl groups, or their heteroaryl analogues or furyl groups. The aryl groups may be in their turn substituted with 1-3 of the following groups: F, Cl, Br, I, OH, SH, NH₂, CHO, COOH, aryl, benzoyl, 1,3-dihydro-1-oxo-2H-isoindolyl-2-yl, 2-thienylcarbonyl, oxyphenyl, oxy-C₁-C₆-alkyl or C₁-C₆-alkyl (linear or branched, comprising 1-2 double bonds or a triple bond), in its turn substituted with 1-3 of the following groups: F, Cl, Br, I, OH, SH, NH₂, CHO, COOH.

Arylalkyl nitriles particularly preferred according to the present invention are mandelonitrile, β-(aminomethyl)-4-chlorobenzene-propionitrile, α-phenyl-2-piperidyl-acetonitrile, α-phenyl-2-piridyl-acetonitrile, 2-(3-benzoylphenyl)propionitrile, α-methyl-3-phenoxybenzene-acetonitrile, 2-fluoro-α-methyl[1,1'-diphenyl]-4-acetonitrile, 4-(1,3-dihydro-1-oxo-2H-isoindolyl-2-yl)-α-methylbenzene-acetonitrile, α-methyl-4-(2-methylpropyl)benzene-acetonitrile, α-methyl-4-(2-thienylcarbonyl)benzene-acetonitrile.

Amongst the aromatic nitriles, the preferred aromatic nitriles according to the present invention are constituted by a nitrile group substituted by an aryl group which may be homocyclic or heterocyclic comprising 4-14 carbon atoms and, if necessary, 1-3 heteroatoms selected from O, S, N. Amongst the aryl groups are to be mentioned for example phenyl and naphthyl groups or their heteroaryl homologues. The aryl group may be in its turn substituted by 1-3 of the following groups: F, Cl, Br, I, OH, SH, NH₂, CHO, COOH, aryl, or C₁-C₆-alkyl (linear or branched, comprising 1-2 double bonds or a triple bond), in its turn substituted by 1-3 of the following groups: F, Cl, Br, I, OH, SH, NH₂, CHO, COOH.

The aromatic nitriles particularly preferred according to the present invention are benzonitrile, 2-furylnitrile, and 2-piridylnitrile.

Further substrates convertible by the biocatalysts of the present invention are amides with the formula R-CONH₂, in which R constitutes an organic residue. Aliphatic amides are preferred in which R is an aliphatic group, the arylalkyl amides in which R is an arylalkyl group and the aromatic amides in which R is an aromatic group.

Amongst the aliphatic amides, the preferred aliphatic amides according to the present invention are these in which R is a linear, branched or cyclic aliphatic group and comprises 1-20 carbon atoms and optionally 1-2 heteroatoms selected from O, S, N. R may be saturated or contain 1-4 double bonds and/or 1-3 triple bonds. Furthermore, R may be substituted by 1-5 of the following groups: F, Cl, Br, I, OH, SH, NH₂, CHO, COOH, cyclopropyl, cyclobutyl, cyclopentyl, cyclohexyl, cycloheptyl. In the case of the presence of cycloaliphatic groups, the latter may contain 1-2 heteroatoms selected from O, S, N, they may contain 1-2 double bonds and may be in turn substituted by 1-3 of the following groups: F, Cl, Br, I, OH, SH, NH₂, CHO, COOH or C₁-C₆-alkyl (linear or branched, comprising 1-2 double bonds or a triple bond), in turn substituted by 1-3 of the following groups: F, Cl, Br, I, OH, SH, NH₂, CHO, COOH.

The aliphatic amides particularly preferred according to the present invention are 2-piperidylamide, 2-methylbutyramide, 2-methylpentanoamide, 3-methyl-pentanoamide, 2-methylhexanamide.

Amongst the arylalkyl amides are preferred for example α-methyl aryl acetamide (or "arylpropionamides"), especially for example the amides which (-through the formal replacement of CONH₂ with COOH-) are the precursors of the class of pharmaceutical compounds denominated profens. A series of arylalkyl amides particularly preferred according to the present invention and constituted by aliphatic amides as above substituted in their turn by 1-4 aryl ("aromatics") or heteroaryl ("heteroaromatics") groups comprising 4-14 carbon atoms and, optionally, 1-3 heteroatoms selected from O, S, N. Amongst the aryl groups are to be mentioned for example the phenyl and naphthyl groups or their heteroaryl homologues or the furyl group. The aryl groups can be in their turn substituted by 1-3 of the following groups: F, Cl, Br, I, OH, SH, NH₂, CHO, COOH, aryl, benzoyl, 1,3-dihydro-1-oxo-2H-isoindolyl-2-yl, 2-thienylcarbonyl, oxy-C₁-C₆-alkyl, oxyphenyl, or C₁-C₆-alkyl (linear or branched, comprising 1-2 double bonds or a triple bond), in its turn substituted by 1-3 of the following groups: F, Cl, Br, I, OH, SH, NH₂, CHO, COOH. Arylalkyl amides particularly preferred according to the present invention are mandelamide, β-(aminomethyl)-4-chlorobenzenpropioamide, α-phenyl-2-piperidyl-acetamide, α-phenyl-2-piridyl-acetamide, 2-(3-benzoylphenyl)propione-amide, α-methyl-3-phenoxybenzene-acetoamide, 2-fluoro-a-methyl[1,1'-diphenyl]-4-acetoamide, 4-(1,3-dihydro-1-oxo-2H-isoindolyl-2-yl)-α-methylbenzene-acetamide, α-methyl-4-(2-methylpropyl)benzene-acetamide, α-methyl-4-(2-thienylcarbonyl)benzene-acetamide. Amongst the aromatic amides, the preferred aromatic amides according to the present invention are constituted by an amidic group substituted by an aryl group that may be homocyclic or heterocyclic comprising 4-14 carbon atoms and, if necessary, 1-3 heteroatoms selected from O, S, N. Amongst the aryl groups are to be mentioned for example phenyl and naphthyl groups or their heteroaryl homologues. The aryl group may be in its turn substituted by 1-3 of the following groups: F, Cl, Br, I, OH, SH, NH₂, CHO, COOH, aryl, or C₁-C₆-alkyl (linear or branched, comprising 1-2 double bonds or a triple bond), in its turn substituted by 1-3 of the following groups: F, Cl, Br, I, OH, SH, NH₂, CHO, COOH.

The aromatic amides particularly preferred according to the present invention are benzamide, 2-furylamide, and 2-piridylamide.

Further substrates convertible by the biocatalyst of the present invention are dinitriles with the general formula NC-R-CN in which R constitutes an organic residue.

According to a preferred embodiment of the present invention, dinitriles are preferred in which R constitutes a linear, branched or cyclic alkylene group and comprises 1-20 carbon atoms and optionally 1-2 heteroatoms selected from O, S, N. R may be saturated or contain 1-4 double bonds and/or 1-3 triple bonds. Furthermore, R may be substituted by 1-5 of the following groups: F, Cl, Br, I, OH, SH, NH₂, CHO, COOH, cyclopropyl, cyclobutyl, cyclopentyl, cyclohexyl, cycloheptyl, aryl. In the case of the presence of cycloaliphatic groups, the latter may contain 1-2 heteroatoms selected from O, S, N, may contain 1-2 double bonds and may be in turn substituted by 1-3 of the following groups: F, Cl, Br, I, OH, SH, NH₂, CHO, COOH or C₁-C₆-alkyl (linear or branched, comprising 1-2 double bonds or a triple bond), in turn substituted by 1-3 of the following groups: F, Cl, Br, I, OH, SH, NH₂, CHO, COOH. In the case of the presence of aryl groups, the latter may contain 4-14 carbon atoms and, if necessary, 1-2 heteroatoms selected from O, S, N, and may be in turn substituted by 1-3 of the following groups: F, Cl, Br, I, OH, SH, NH₂, CHO, COOH or C₁-C₆-alkyl (linear or branched, comprising 1-2 double bonds or a triple bond), in its turn substituted by 1-3 of the following groups: F, Cl, Br, I, OH, SH, NH₂, CHO, COOH. Preferred aryl groups are constituted by phenyl, naphthyl and piridyl.

According to a further embodiment of the present invention, are preferred dinitriles in which R constitutes an aryl group ("arylene") which can be homocyclic or heterocyclic comprising 4-14 carbon atoms and, if necessary, 1-3 heteroatoms selected from O, S, N. Amongst the aryl groups are to be mentioned for example the phenylene, naphthylene groups, or their heteroaryl homologues. The Aryl group may be in its turn substituted by 1-3 of the following groups: F, Cl, Br, I, OH, SH, NH₂, CHO, COOH, aryl, or C₁-C₆-alkyl (linear or branched, comprising 1-2 double bonds or a triple bond), in its turn substituted by 1-3 of the following groups: F, Cl, Br, I, OH, SH, NH₂, CHO, COOH.

Amongst the particularly preferred dinitriles according to the present invention are to be named 2-methylphenylmalononitrile and 1,3-benzodinitrile.

A conversion using the biocatalyst of the present invention is the conversion of nitriles into their respective acids. For this conversion, the aliphatic nitrile, arylalkyl nitrile or aryl nitrile substrate as above is reacted with the biocatalyst in a buffered solution as above, verifying the formation of the desired acid with appropriate analytical methods. The reaction is interrupted by centrifugation and/or decanting of the biocatalyst following the achievement of the acid yield desired, and the acid is isolated. The times and the temperatures of the reaction vary according to the specific substrate chosen and can be adequately optimised by experts in the field so as to obtain, through the nitrile hydratase and amidase activities, -for at least a fraction equal to 20% of the initial nitrilic substrate-complete hydrolysis of the nitriles to give the respective acid.

A further conversion using the biocatalyst of the present invention is the conversion of nitriles into their respective amides. For this conversion, the aliphatic nitrile, arylalkyl nitrile or aryl nitrile substrates as above are reacted together with the biocatalyst in a buffered solution as above verifying the formation of the desired amides with the appropriate analytical methods. The reaction is interrupted by centrifugation and/or decanting of the biocatalyst after achievement of the desired amide yield, and the amide is isolated. The times and the temperatures of reaction vary according to the specific substrate chosen and can be adequately optimised by experts in the field so as to make prevail the nitrile hydratase activity, so as to obtain an amidic fraction equal to at least 15% of the initial nitrilic substrate. According to a preferred embodiment, to that end, it is possible for example to inhibit the amidase activity of the biocatalyst through the addition of diethylphosphoramidate to the reaction mixture.

A further conversion utilising the biocatalyst of the present invention is the conversion of amides into their respective acids. For this conversion, the aliphatic amide, arylalkyl amide or aryl amide substrate as above is reacted together with the biocatalyst in a buffered solution as above, verifying the formation of the desired acid with the appropriate analytical methods. The reaction is interrupted by centrifugation and/or decanting the biocatalyst after achievement of the desired acid yield and the acid is isolated. The times and the temperatures of the reaction vary according to the specific substrate chosen and can be adequately optimised by experts in the field.

A further conversion utilising the biocatalyst of the present invention is the regioselective hydratation or hydrolysis of nitriles to give the respective mononitrile-monoamide and/or mononitrile monoacid and/or monoamide-monoacid-derivatives. For this conversion, the dinitrile substrate as above is reacted with the biocatalyst in a buffered solution as above, verifying the formation of the desired mononitrile-amidic and/or mononitrile acidic and/or monoamide-monoacid derivative with the appropriate analytical methods. The reaction is interrupted by centrifugation and/or decanting of the biocatalyst after achievement of the desired yield of the product, and the product is isolated. The times and the temperatures of the reaction vary according to the specific substrate chosen and can be adequately optimised by experts in the field.

According to a preferred embodiment of the present invention, the chiral substrates (nitrile, amide or dinitrile) in racemic form are subjected to conversion as above using the biocatalyst according to the invention. The enantioselectivity of the biocatalysts according to the present invention determines the preferred conversion of one of the two enantiomers contained in the racemic substrate. For example, it has been found during the course of experimentation carried out by the inventors that the biocatalysts obtained from the micro-organism *Agrobacterium radiobacter 30"60* NCIMB 41108, particularly preferred in the field of the present invention, show a marked S-enantioselectivity for both enzymatic activities, that is the nitrile hydratase and amidase activities. As is usual for biocatalysts, the displayed enantioselectivity depends slightly on the age of a given catalyst culture. Such effect, according to which the overall chemical conversion yield decreases with a given culture's age whereas its enantioselectivity increases instead in time, is largely known to the skilled man who will be able to select the desired optimum according to his specific needs. On top of that, the enantioselectivity may further be fine-tuned in both, in yield (e.e.) and, to some extent, even in preference for a specific enantiomeric form (R or S), from case to case, through the use of specifically selected inducers depending on the chosen substrate. In the present case, as shown hereinafter, the marked S-enantioselectivity of *Agrobacterium radiobacter 30"60* NCIMB 41108 has been confirmed for the overwhelming majority of the substrate/inducer-combinations tested.

According to a further preferred embodiment of the present invention, prochiral dinitrile substrates are subjected to the conversions as above utilising the biocatalyst according to the invention.

### Enantioselectivity for the S-enantiomer.

According to a preferred embodiment, the enantioselective conversion of racemic forms of nitriles to give the respective S-acids is carried out in a single reaction comprising two subsequent phases: one phase a) of the enantioselective conversion of racemic nitriles to S-amides and a phase b) of the enantioselective conversion of S-amides to S-acids. Since the two phases a) and b) are also active independently of one another, two further aspects of the invention are constituted respectively by: a') a process for the enantioselective conversion of racemic nitriles to give the S-amides and b') a process for the conversion of racemic amides to give S-acids, characterised by the fact of using the micro-organisms according to the invention. It should be noted that in the case of the conversion of racemic nitriles to give the respective S-acids, a percentage of R-amide is frequently also isolatable which, although it is formed much more slowly in the first phase a) under the influence of the nitrile hydratase activity (specific, as pointed out, for the S-nitrile), once formed is not further processed by the amidase in phase b) which is specific for the S-amide which is converted into the S-acid. It is to be noted however that in cases in which it is desired to specifically isolate the R-amide with high yield, it is favourable to start with the racemic amide as substrate (phase b')) and therefore recover the non-processed R-enantiomer following formation of the S-acid. The process of enantioselective conversion of racemic nitriles to their respective S-acids via the respective S-amides uses nitriles in racemic form as substrates, selected from: aryl-alkyl nitriles and aliphatic nitriles as defined above. Particularly preferred amongst the aryl-alkyl nitriles in the conversion reaction comprising the two phases a) and b) are the α-methyl aryl acetonitriles ("propionitriles"). Belonging to this class are the racemic nitrile precursors of the profens, molecules of notable pharmaceutical interest in enantiomerically pure form. Therefore, according to this particularly preferred aspect, the invention is concerned with processes for the enantioselective preparation of S-α-methyl-3-phenoxibenzene-acetic acid (S-fenoprofen), of S-2-fluoro-α-methyl[1,1'-diphenyl]-4-acetic acid (S-flurbiprofen), of S-4-(1,3)-dihydro-1-oxo-2H-isoindol-2yl)-α-methylbenzene-acetic acid (S-indoprofen), of S-α-methyl-4-(2-thienylcarbonyl)benzene-acetic acid (S-suprofen), of S-α-methyl-4-(2-methylpropyl)benzene-acetic acid (S-ibuprofen).

In particular it has been observed that the enantioselective conversion reaction, in particular of the α-methyl-arylacetonitriles shows an optimum temperature at temperatures comprised of between 38 and 42°C: under these conditions the yield of conversion of the acid in enantiomerically pure form is particularly advantageous.

It is to be further noted that in the conversion reaction according to the phase b') amongst the aryl-alkyl amides, the α-methyl aryl acetamides ("propionamides") are particularly preferred. Belonging to this class are the racemic amides, precursors of profens, molecules of notable pharmaceutical interest in enantiomerically pure form. Therefore, according to this particularly preferred aspect, the invention is concerned also with processes for the enantioselective preparation of S-α-methyl-3-phenoxibenzen-acetic acid (S-fenoprofen), of S-2-fluoro-α-methyl[1,1'-diphenyl]-4-acetic acid (S-flurbiprofen), of S-4-(1,3)-dihydro-1-oxo-2H-isoindol-2yl)-α-methylbenzeneacetic acid (S-indoprofen), of S-α-methyl-4-(2-thienylcarbonyl)benzeneacetic acid (S-suprofen), of S-α-methyl-4-(2-methylpropyl)benzeneacetic acid (S-ibuprofen) which are based on the use of the relative amidic precursors as substrates.

In the conversion reaction of racemic nitriles to S-acids and in that of racemic amides to S-acids, the aliphatic precursors (nitriles or amides) are also particularly preferred. Amongst the aliphatic nitriles as above and amongst the aliphatic amides as above, are particularly preferred these which are precursors of acids or related esters utilisable as flavouring agents such as 2-methylbutyrric acid (which gives a fruit flavour), 2-methylpentanoic acid (which gives a cheese flavour), the methyl ester of 2-methylpentanoic acid (which is used in oven cooked foods, meat products and sweets), the ethyl ester of 2-methylpentanoic acid (which gives the taste of pineapple or apple), the ethyl ester of 3-methylpentanoic acid (which gives the taste of pineapple or apple) and 2-methylhexanoic acid (which gives the flavour of cheese or tropical fruits) and which in the past, for reasons of simplicity, were frequently used in racemic forms. The present invention therefore makes available an elegant access into the enantioselective synthesis of flavouring agents (or, in the case of the respective esters, their precursors) as above. It has further been observed that the micro-organisms according to the invention are capable of converting in a regioselective (and preferably enantioselective) manner dinitriles as above to give the respective mononitrile-monoamide and/or mononitrile acid and/or monoamide-monoacid derivatives. For this reason, according to one of its further aspects, the invention relates to a process for the regioselective conversion (preferably enantioselectively) of dinitriles of the general formula as defined above.

### EXPERIMENTAL PART.

### Example 1. Mutagenesis of the parent strain.

The parent strain, characterised as *Agrobacterium radiobacter,* was isolated from a crude bacterial extract with antibiotic activity. In order to determine activity towards nitrilic substrates, it was sub-cultured in medium containing butyronitrile and after some passages and induction with valeronitrile, a weak nitrile-hydratase/amidase activity was measured on benzoylphenylpropionitrile (BPN) of the wild type strain as such selected. To obtain a semi-constitutive strain, an aliquot of wild type parent strain was subjected to a treatment of mutagenesis, constituted by two treatment cycles with ethylmethanesulphonate, one cycle with nitrosoguanidine and two of mutagenesis with UV irradiation.

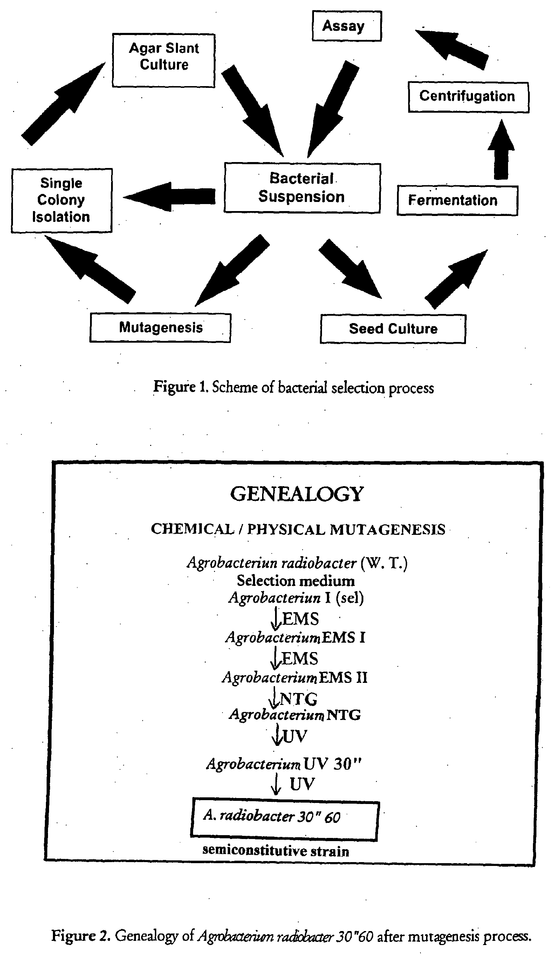

At each stage of mutagenesis, an aliquot of the culture was assayed for the nitrile conversion activity (Figure 1).

The mutagenesis protocols were adapted to *Agrobacterium radiobacter* from Ausubel, F.M. et al. "Current Protocols in Molecular Biology" Wiley Interscience Editions **vol. 2,** section. 13.3.1, 1994 and are briefly reported here for completeness.

### 1) Treatment with Ethylmethanesulphonate (EMS).

A culture grown to saturation was centrifuged and washed twice with potassium phosphate buffer 0.05 M, pH 7.2. To aliquots of 1.7 ml of bacteria in sterile buffer (approx. 6.6 x 10⁷ cells) was added 50 µl of EMS and incubated at 30°C for 1 hour with agitation. 200 µl of the mutagenised bacterial suspension was added to 8 ml of a solution of 5% Na₂S₂O₃ to block the reaction. The bacteria were then centrifuged and washed twice to remove the mutagenic agent.

### 2)Treatment with nitrosoguanidine (N-methyl-N'-nitro-N-nitrosoguanidine, NTG).

A culture grown to saturation was centrifuged and washed twice with potassium phosphate buffer 0.05 M, pH 7.2. To aliquots of 1.7 ml of bacteria in sterile buffer (approx. 6.6 x 10⁷ cells), were added 150 µg/ml of NTG and incubated at 30°C for 1 hour with agitation. 200 µl of the mutagenised bacterial suspension was added to 8 ml of a solution of 5% Na₂S₂O₃, to block the reaction. The bacteria were then centrifuged and washed twice to remove the mutagenic agent.

### 3)Treatment with UV.

A culture grown to saturation was centrifuged and washed twice with potassium phosphate buffer 0.05 M, pH 7.2. After centrifugation, the bacteria (approx. 2 x 10⁸) were resuspended in 5 ml of sterile double distilled H₂O and subjected to UV irradiation at 254 nm, using 300 ergs/mm². The irradiation time varied between 15" and 90". Under these conditions the survival of the bacterial culture varied from 40 to 70%.

The complete mutagenesis treatment, comprised of 5 steps, that is it is comprised of two steps of treatment with EMS, one with NTG, in addition to two irradiations with UV, the first for 30 seconds, and the second for 60 seconds, and represented schematically in Figure 2.

To select the nitrile hydratase/amidase activity, around 100 bacteria/ plate were made to grow utilising nitrilic or amidic compounds as sole nitrogen and minimal carbon sources. Approx. 200 colonies were isolated and assayed for nitrile conversion activity.

In Figure 1 is reported the complete treatment process with which was obtained the semi-constitutive strain 41108 possessing nitrile conversion activity, denominated *Agrobacterium radiobacter 30"60,* and deposited with the authorised centre NCIMB (23

St. Machar Drive, Aberdeen AB24 3RY, Scotland, UK), on 3rd of May 2001.

### Example 2. Characterisation of the semi-constitutive strain Agrobacterium radiobacter 30"60 n° 41108.

The characteristics relating to the isolated strain are summarised in table 1 and are hereby further briefly commented as follows.

### Morphological characteristics.

The strain *Agrobacterium radiobacter* 30"60 is present as a rod of approx. 0.8 µm by 1.5-3 µm. Under the microscope it appears mobile, does not form spores and is Gram-negative after appropriate staining. Starting from lyophilised strain, after 48 hours of growth on solid medium (seed-agar) the strain forms rough, rounded colonies, of 1.2 mm in diameter and of ivory/white colour.

*Culture and growth properties.* The strain represents an obligatory aerobe and does not reduce nitrates. It is oxidase negative and catalase positive, contrary to the parental strain. The strain grows at a pH comprised of between 6-9 and at a temperature comprised of between 5 and 45°C. It does not produce acid from: glycerol and from the following sugars: glucose, fructose, maltose, sucrose, sorbitol, mannitol, lactose, starch. It does not reduce nitrates, does not produce indole or hydrogen sulphide, nor produce gas from a single sugar. It utilises inorganic nitrogen sources and mannose, fructose, galactose, maltose, lactose, starch, gluconate, malate and succinate as sole carbon sources. Caprate, adipate and citrate are not utilised.

**Table 1.** Morphological, cultural and physiological characteristics of the bacterium *Agrobacterium radiobacter* 30"60 (NCIMB 41108) according to the classification API 20 NE (% id = 99,6; T = 0,72; 99% - very good identification).

**Table 1**

| Microbiological characteristics | *A. radiobacter* 30" 60 (41108) | *Wild-type* |
|---|---|---|
| **Morphology** | Rod shape 0.8 x 1.5-3.0 µm | Rod shape 0.8 x 1.5-3.0 µm |
| Motility | + | + |
| Spore formation | - | - |
| Gram staining | - | - |
| **Culture** | | |
| Appearance | Round, rough and opaque | Convex and smooth |
| Colour | Ivory white | Reddish |
| Diameter | 1-2 mm | 4 mm |
| **Physiological** | | |
| Oxygen requirement | Obligatory aerobe | Obligatory aerobe |
| Growth pH | 6-9 | 6-9 |
| Growth temperature | 5-45° | 5-45° |
| Reduction of nitrates to nitrites | - | - |
| Reduction of nitrites to nitrogen | - | - |
| Production of indole | - | - |
| Production of acids | - | - |
| Arginine dihydrolase | - | - |
| Urease | -/+ | + |
| β-glucosidase | + | + |
| Proteases | - | - |
| β-galactosidase | + | + |
| Glucose | Assimilated | Assimilated |
| Arabinose | Assimilated | Assimilated |
| Mannose | Assimilated | Assimilated |
| Mannitol | Assimilated | Assimilated |
| N-acetyl-glucosamine | Assimilated | Assimilated |
| Maltose | Assimilated | Assimilated |
| Lactose | Assimilated | Assimilated |
| Starch | Assimilated | Assimilated |
| Gluconate | Assimilated | Assimilated |
| Caprate | - | - |
| Adipate | - | - |
| Malate | Assimilated | Assimilated |
| Citrate | - | - |
| Phenyl-acetate | - | - |
| Oxidase | - | + |
| Catalase | + | - |

### Example 3. Preparation of the bacterial biocatalyst of the mutagenised strain NCIMB 41108.

The enzymatic activity was evaluated using a bacterial pellet obtained after fermentation for 48 hours in fermentation medium as described below. Briefly, the culture was amplified from a single colony to begin with in a small volume of inoculating medium and then, for preparative purposes, in 100 ml of fermentation medium.

*Inoculating medium:* peptone 10 g/L, yeast extract 10 g/L, NaCl 5g/L, glucose 5g/L. The pH of the medium was adjusted to pH 7.2 with NaOH and sterilised.

*Fermentation medium:* MgSO₄ (0.5 g l⁻¹), ,CaCl₂.6H₂O (0.1 g l⁻¹), FeSO₄ (0.4 g l⁻¹), CoCl₂ (0.1 g l⁻¹), MnSO₄ (0.01 g l⁻¹), (NH₄)₂SO₄ (5 g l⁻¹), Yeast Extract (10 g l⁻¹), Glucose (5 g l⁻ ¹). The inducer, or the inducers, were added to the fermentation medium alone or in combination, sterilely, to a final concentration comprised of between 0.5-5 g/L. In particular, if not stated differently, valeronitrile, N-butyronitrile, isobutyronitrile and ε-caprolactam were used at a concentration respectively of 2.5 g/l and added in a single solution at the start of the process. To measure the basal activity of the strain 41108, no inducer was added to the fermentation medium.

*Pre-inoculation.* Was performed from a bacterial slant in agar in 50 ml of inoculation medium, in a 250 ml flask and grown at 30°C, for 16-24 hours at 180 rpm.

*Inoculation.* 10 ml of the pre-inoculation, was inoculated in 100 ml of fermentation medium containing or lacking inducer, in a 0.5L flask and grown at 30°C, for 24-72 hours, with agitation at 300 rpm. (if conducted on a lager scale, for example in a 101 fermenter, aeration was effected at a pressure of 0.5 Kg/cm² and at a flow rate of 1.0 vvm with dried, filter-sterilised air) The bacteria were recovered by centrifugation and the bacterial precipitate (*pellet*) washed twice with isotonic saline and maintained at 4°C until the time of use (wet bacterial pellet or bacterial paste). This bacterial precipitate constitutes the bacterial biocatalyst.

### Example 4. Characterisation of the enzymatic activity of strain NCIMB 41108.

### Analytical methods.

*Reaction scheme.* To the cellular paste (250 mg, unless indicated otherwise) in phosphate buffer 35 mM, pH 7.7 (5 ml, unless indicated otherwise), containing Triton X-100 or Tween 80 at a final concentration of 2.66%, was added the substrate solution dissolved in methanol in a volume equal to 5-10% of the final volume (250 µl, unless indicated otherwise) and the reaction mixture incubated at 30°C or at other temperatures, as indicated in the examples. At preset time intervals, aliquots of the mixture were sterilely removed with a syringe, using a Minisart RC 25 filter 0,22 µm (Sartorius, Germany) and directly analysed by HPLC. The enzymatic activity was determined by the mean activity measured in the initial, linear phase of the conversion curve, relative to the wet weight of the bacterial paste. The specific activity U (µmol min⁻¹) per gram of wet weight was indicated as IU g⁻¹.

*Evaluation of the conversion percentages and of the enantiomeric excess.* The percentage conversion of the substrate was measured by reverse phase HPLC using a reverse phase column of dimensions: 250 x 4.6 mm; 10 µm (Lichrosorb 10 RP18), and phosphate buffer at various percentages (10 mM, pH 2.5 e 3.5) with acetonitrile at 40 or 50% depending on the molecule to be detected. A flow rate of 1.0 ml min⁻¹ was used, and a detection wavelength of 220-254 nm. The e.e. values of the reaction products obtained from benzoylphenylpropionitrile were determined by HPLC fitted with a chiral column (dimensions: 100 x 4 mm; Chromtech AGP-column) and phosphate buffer used as eluent (10 mM, pH 7.0) with isopropanol (1%, v/v) and dimethyloctylamine (0,02%, v/v). Enantiomeric excess of α-phenylpropionic acid was determined by HPLC on the same column (100 x 4 mm; Chromtech AGP-column), using phosphate buffer as eluent (20 mM, pH 5.0,) with dimethyloctylamine (0.04%, v/v). The e.e. values of the reaction products obtained from α-methyl-4-(2-thienylcarbonyl)-benzene-acetonitrile were determined by HPLC fitted with a chiral column (dimensions: 100 x 4 mm; Chromtech AGP-column) and potassium phosphate buffer used as eluent (20 mM, pH 7.0) with dimethyloctylamine (0,1%, v/v). The e.e. values of the reaction products obtained from α-methyl-3-phenoxybenzene acetonitrile were determined by HPLC fitted with a chiral column (dimensions: 100 x 4 mm; Chromtech AGP-column) and phosphate buffer used as eluent (100 mM. pH 7.0) with isopropanol (1 %. v/v). The e.e. values of the reaction products obtained from 2-fluoro-α-methyl [1,1'-diphenyl]-4-acetonitrile were determined by HPLC fitted with a chiral column (dimensions; 100 x 4 mm; Chromtech AGP-column) and using phosphate buffer as eluent (10 mM, pH 6.5) with isopropanol (5 %, v/v) and dimethyloctylamine (0.02 %. v/v). The e.e. values of the reaction products obtained from α-methyl-4-(2-methylpropyl)benzene-acetonitrile and from α-methyl-4-(2-methylpropyl)benzene-acetamide were determined by HPLC fitted with a chiral column (dimensions: 100 x 4 mm; Chromtech AGP-column) and phosphate buffer used as eluent (10 mM, pH 7.0) with dimethyloctylamine (0.02 %, v/v). The e.e. values of aliphatic nitriles were determined by gas-chromatography on a Mega system with the following characteristics: column dimensions 25 m x 0.25 mm; hydrogen 0.4 bar; starting temperature: 40 °C, isotherm 5 min, 1.5 °C/min for 180 °C. Injection temperature: 220 °C; detection temperature 250 °C; phase: diacetyl tert-butylsilyl-β-cyclodextrin (OV1701).

### Example 5. Use of the bacterial biocatalyst for the conversion of arylalkyl nitriles (2-(3-benzoylphenyl)propionitrile and α-methyl-4-(2-thienylcarbonyl)benzene-acetonitrile) to give ketoprofen and suprofen.

### 5.1. Production of S-(+)-Ketoprofen from benzoyl-phenyl-propionitrile in basal conditions utilising the bacterial pellet of strain NCIMB 41108 (non-induced) as biocatalyst.

With the aim of determining what would be the yield and the enantiomeric specificity in basal conditions (constitutive enzymatic activity) the bacterial pellet of non-induced strain 41108 was used, according to the reaction scheme indicated in example 4: to 50 mg of benzoylphenylpropionitrile in 250 µl of methanol, were added 5 ml of KH₂PO₄ buffer, pH 7.7, containing 2.66% of Triton X-100 and 250 mg of wet bacterial pellet, non-induced, prepared as described in example 3. The solution was incubated at 30°C with agitation. After 17 hours of reaction the HPLC analysis showed the presence of benzoylphenyl propionamide (2%) and of S-(+)-benzoylphenyl propionic acid (S-ketoprofen) (8%, e.e. > 99%) and an enzymatic activity of 0.08 IU g⁻¹ of wet cells.

### 5.2. Production of S-(+)-Ketoprofen from benzoyl-phenyl-propionitrile utilising the bacterial pellet of strain NCIMB 41108 induced with valeronitrile, as biocatalyst.

The reaction scheme described in example 4 was used: to 50 mg of racemic benzoylphenylpropionitrile in 250 µl of methanol, were added 5 ml of KH₂PO₄ buffer, pH 7.7, containing 2.66% of Triton X-100 and 250 mg of wet bacterial pellet, derived from cells induced with valeronitrile 0,25%, prepared as described in example 3. The solution was incubated at 30°C with agitation. After 17 hours of reaction the HPLC analysis showed the total conversion of substrate and the presence of S-(+)-benzoylphenyl propionamide (55%) and of S-(+)-benzoylphenyl propionic acid (S-ketoprofen) (45%, e.e. > 96 %) and an enzymatic activity of 0.83 IU g⁻¹ of wet cells.

### 5.3. Production of S-(+)- "ketoprofenic amide " from benzoyl-phenyl-propionitrile using the bacterial pellet of strain NCIMB 41108 induced with valeronitrile as biocatalyst.

The enzymatic activity was measured after reaction carried out according to the scheme reported in example 4: to 50 mg of racemic benzoylphenylpropionitrile in 250 µl of methanol, were added 5 ml of KH₂PO₄ buffer, pH 7.7, containing 2.66% of Triton X-100 and 250 mg of *wet bacterial pellet,* derived from cells induced with valeronitrile 0,25%, prepared as described in example 3. The solution was incubated at 30°C for 1 hour. Following this time, the HPLC showed the presence of S-benzoylphenyl propionamide (40%, e.e. >95%) and an enzymatic activity equal to 5.66 IU g⁻¹ of wet cells.

### 5.4. Effect of temperature on the conversion of benzoyl-phenyl-propionitrile utilising the bacterial pellet of strain NCIMB 41108 induced with valeronitrile as biocatalyst.

To compare the effect of temperature on enzymatic activity, the experiment was repeated in a way equal to that described in the preceding paragraph, except for the temperature of the reaction which was 40°C. The detection of the reaction products was performed after 1 hour and after 17 hours. After 1 hour, the HPLC showed the presence of S-(+)-benzoylphenyl propionic acid (6%, e.e.>99%), of S-(+)-benzoylphenyl propionamide (30%, e.e.>95%) and benzoylphenylpropionitrile (64%). After 17 hours, the HPLC showed the presence of S-(+)-2-(3-benzoylphenyl)propionic acid (S-(+)-ketoprofen) (44%, e.e. >98%) and benzoylphenylpropionitrile (56%). The data demonstrate that at 40°C, the amidase activity is greater than at 30°C. This characteristic, further to relating to the temperature, is bound to the availability of the sole enantiomer of preference and this is thanks to the raised enantioselectivity that the nitrile hydratase acquires at this temperature.

### 5.5. Effect of the induction with valeronitrile and ε-caprolactam.

The experiment was repeated utilising total bacterial extract, in the same reaction conditions already described, at 30°C for 17 hours, with bacterial pellet induced with valeronitrile and ε-caprolactam (0,25% each) in the fermentation phase. After this period, the HPLC analysis showed benzoylphenyl propionamide (51%) and S-(+)-benzoylphenyl propionic acid (48%, e.e. >80%). The data demonstrated that the enantioselective efficiency of the reaction can be modified by the structure of the inducing molecule and/or by the association of more molecules with inducing functions, in addition to the substrate that is intended for processing.

### 5.6. Effect of induction with isobutyronitrile.

The experiment was repeated using as total bacterial extract, in the same reaction conditions already described, at 30°C for 24 hours, the bacterial *pellet* induced with isobutyronitrile (0.1 %) in the fermentation phase. Following this period the HPLC analysis showed benzoylphenylpropionitrile (56%), benzoyl phenyl propionamide (4%) and S-(+)-benzoylphenylpropionic acid (40%, e.e. 90%). The data showed again that the enantioselective efficiency of the reaction and the recovered products can be modulated by the structure of the inducer molecule and/or by the association of other molecules with inducer functions in addition to and according to the substrate intended for processing.

### 5.7. Production of R-(-)-Suprofen from a-methyl-4-(2-thienylcarbonyl)benzene-acetonitrile using as biocatalyst the bacterial pellet of strain NCIMB 41108 induced with isobutyronitrile

The reaction scheme described in example 4 was used: to 50 mg of racemic α-methyl-4-(2-thienylcarbonyl)benzene-acetonitrile in 250 µl of methanol, 5 ml of KH₂P0₄ buffer pH 7.7, containing 2.66% Triton X-100 and 250 mg of wet bacterial pellet, derived from cells. induced with isobutyronitrile (0.2%), prepared as described in example 3, were added. The solution was incubated at 30°C with agitation. Following 28 hours of reaction, the HPLC analysis showed the presence of α-methyl-*4*-(2-thienylcarbonyl)benzene-acetamide (1%), of R-(-)-α-methyl-4-(2-thionylcarbonyl)benzene-acetic acid (R-Suprofen) (42%, e.e.>98%) and of α-methyl-4-(2-thienylcarbonyl)benzene-acetonitrile and an enzymatic activity of 0.21 IU g⁻¹ of wet cells.

### 5.8. Production of R-(-)-Suprofen from α-methyl-4-(2-thienylcarbonyl)benzene-acetonitrile using as biocatalyst the bacterial pellet of strain NCIMB 41108 induced with ε-caprolactam.

The reaction scheme described in example 4 was used: to 50 mg of racemic α-methyl-4-(2-thienylcarbonyl)benzene-acetonitrile in 250 µl of methanol, 5 ml of KH₂P0₄ buffer pH 7.7, containing 2.66% Triton X-100 and 250 mg of wet bacterial pellet, derived from cells induced with ε-caprolactam (0.2%), prepared as described in example 3, were added. The solution was incubated at 30°C with agitation. Following 5 hours of reaction, the HPLC analysis showed the presence of α-methyl-*4*-(2-thienylcarbonyl)benzene-acetamide (1%), of R-(-)-α-methyl-4-(2-thionylcarbonyl)benzene-acetic acid (R-Suprofen) (47%, e.e.>98%) and of α-methyl-4-(2-thienylcarbonyl)benzene-acetonitrile and an enzymatic activity of 1.30 IU g⁻¹ of wet cells.

### 5.9. Effect of induction with valeronitrile.

Using the reaction scheme described in example 4: to 50 mg of racemic *a-methyl-4-(2-thienylcarbonyl)benzene-acetonitrile* in 250 µl of methanol, were added 5 ml of KH₂P0₄ buffer, pH 7.7, containing 2.66% Triton X-100 and 250 mg of wet bacterial pellet, derived from cells induced with valeronitrile 0,2%, prepared as described in example 3. The solution was incubated at 30°C with agitation. Following 2 hours of reaction, the HPLC analysis show the presence of α-methyl-4-(2-thienylcarbonyl)benzene-acetamide (0.5%), of S-α-methyl-4-(2-thienylcarbonyl)benzene-acetic acid (S-Suprofen) (13,6%, e.e. ≤55%) and of α-methyl-4-(2-thienylcarbonyl)benzene-acetonitrile (86,4%), and an enzymatic activity of 1.87 IU g⁻¹ of wet cells. Thus, in comparison to examples 5.7 and 5.8, and as discussed previously, in this case it is still more evident that not only the enantioselective efficiency but also the "enantiomeric preference" of the reaction can be modulated by the structure of the inducing molecule and according to the substrate intended for processing.

### Example 6. Production of profens from their corresponding nitriles or amides.

### 6.1. Conversion of fenoprofen precursors utilising the bacterial pellet of strain NCIMB 41108, induced with valeronitrile, as biocatalyst.

To 50 mg of racemic α-methyl-3-phenoxibenzen-acetonitrile in 250 µl of methanol, were added 5 ml of KH₂PO₄ buffer, pH 7.7 and containing 2.66% of Triton X-100 and 250 mg of wet bacterial pellet, derived from cells induced with valeronitrile 0,25% prepared as described in example 3, according to the reaction scheme described. The solution was incubated at 30°C for 1 hour. Following this time the HPLC showed the presence of α-methyl-3-phenoxibenzen-acetoamide (25%) and an enzymatic activity equal to 5.23 IU g⁻¹ of wet cells, of S-α-methyl-3-phenoxibenzen-acetic acid (S-fenoprofen) (10.4%, e.e.>98%) and of nitriles equal to 64%.

### 6.2. Production of S-(+)-Flurbiprofen from 2-fluoro-α-methyl[1,1'-diphenyl]-4-acetonitrile using the bacterial pellet of strain NCIMB 41108, induced with valeronitrile, as biocatalyst.

The reaction scheme in example 4 was used: to 50 mg of racemic *2-fluoro-α methyl [1*,1'-*diphenyl-4-acetonitrile* in 500 µl of methanol, were added 5 ml of KH₂PO₄ buffer, pH 7.7, containing 2.66% of Triton X-100 and 250 mg of wet bacterial pellet, derived from cells induced with valeronitrile 0,2%, prepared as described in example 3. The solution was incubated at 30°C with agitation. After 24 hours of reaction, the HPLC analyses showed the presence of 2-*fluoro*-α-methyl [1,1'-diphenyl]-4*-acetamide* (16%), of S-(+)-2-fluoro-α-methyl[1,1'-biphenyl]-4-acetic acid (S-Flurbiprofen) (27%,e,e. > 96 %) and of 2-*fluoro-α-methyl[1,1'-biphenyl]-4-acetonitrile (57%)* and an enzymatic activity of 0.26 IU g⁻¹ of wet cells.

### 6.3. Production of S-(+)-lbuprofen from a-methyl-4-(2-methylpropyl)benzene-acetamide utilising the bacterial pellet of strain NCIMB 41108, induced with valeronitrile, as biocatalyst.

The reaction scheme described in example 4 was used: to 50 mg of racemic α-methyl-4-(2-methylpropyl)benzene-acetamide in 250 µl of methanol, were added 5 ml of KH₂PO₄ buffer, pH 7.7, containing 2.66% of Triton X-100 and 250 mg of wet bacterial pellet, derived from cells induced with valeronitrile 0,2% prepared as described in example 3. The solution was incubated at 30°C with agitation. After 2 hours of reaction the HPLC analyses showed the presence of α-methyl-4-(2-methylpropyl)benzene-acetamide (60%), of S-(+)-α-methyl-4-(2-methylpropyl)benzene acetic acid (S-lbuprofen) (40%, e.e. ≤ 93 %), and an enzymatic activity of 3.24 IU g⁻¹ of wet cells.

### Example 7. Production of S-(+)-2-phenylpropionic acid from α-methylbenzyl-cyanide utilising the bacterial pellet of strain NCIMB 41108, induced with valeronitrile ed ε-caprolactam, as biocatalyst.

The reaction was carried out according to the reaction scheme described, but varying the substrate/bacterial pellet ratio as follows: to 100 mg of α-methylbenzyl cyanide in 250 µl of methanol, were added 5 ml of KH₂PO₄ buffer, pH 7.7, containing 2.66% of Triton X-100 and 100 mg di *wet bacterial pellet,* induced with valeronitrile and ε-caprolactam 0,25%, prepared as described in example 3. The solution was incubated at 30°C with agitation. After 8 hours of reaction the HPLC analyses showed the presence of phenylpropionamide (55%) and of S-(+)-2-phenylpropionic acid (45%, e.e.=96%) and an enzymatic activity of 15.88 IU g⁻¹ of wet cells.

The data demonstrate that this molecule is transformed at an approximately 20 times higher rate.

### Example 8. Conversion of dinitriles utilising the bacterial pellet of strain NCIMB 41108, induced with valeronitrile, as a biocatalyst.

### 8.1. Production of 2-methyl-phenyl-malononitrile-monoamide from 2-methyl-phenyl-malononitrile.

The reaction was carried out as described in example 4 in the paragraph: *Evaluation of the enzymatic activity:* to 100 mg of methylphenyl malononitrile dissolved in 250 µl of methanol, were added 5 ml of KH₂PO₄ buffer, pH 7.7, containing 2.66% of Triton X-100 and 100 mg wet bacterial pellet, induced with valeronitrile 0,25% prepared as described in example 3. The solution was incubated at 30°C with agitation. After 1.5 hours of reaction the HPLC analyses showed the presence of 2-methylphenylmalononitrile (20%) and of 2-methylphenylmalononitrileamide (80%).

The data demonstrate that the reaction is regioselective.

### 8.2. Production of 4-cyano-3-phenyI-butyramide from 3-phenyl-pentane-dinitrile.

The reaction was carried out as described in example 4 in the paragraph: *Evaluation of the enzymatic activity.* To 100 mg of *3-phenyl-pentandinitrile* dissolved in 250 µl of methanol, were added 5 ml of KH₂PO₄ buffer, pH 7.7, containing 2.66% of Triton X-100 and 500 mg of wet bacterial pellet, induced with valeronitrile 0,2% prepared as described in example 3. The solution was incubated at 30°C and agitated. After 4 hours of reaction, the HPLC analyses showed the presence of *4-cyano-3-phenyl-butyramide* (100%) and the enzymatic activity of 4.89 IU g⁻¹ of wet cells.

The data demonstrate that the reaction is regioselective,

### Example 9. Conversion of aliphatic nitrites utilising the bacterial pellet of strain NCIMB 41108, induced with valeronitrile, as biocatalyst.

### 9.1. Production of S-(+)-2-methyl-pentanoic acid from 2-methyl-pentanonitrile.

The reaction was carried out according to the reaction scheme in example 4, with the following variations: to 100 mg of 2-methyl-pentanonitrile dissolved in 250 µl of methanol, were added 5 ml of KH₂PO₄ buffer, pH 7.7, containing 2.66% of Triton X-100 and 100 mg of wet bacterial pellet induced with valeronitrile 0,25%, prepared as described in example 3. The solution was incubated at 30°C with agitation. After 4 hours of reaction, Gas-chromatograph analysis showed the presence of 2-methyl-pentanoamide (53%) and of S-(+)-2-methyl-pentanoic acid (47%, e.e.=96), with an enzymatic activity of 35.9 IU g⁻¹ of wet cells.

### 9.2. Production of (5)-(+)2-ethyl hexanoic acid from 2-ethylhexane nitrile.

The reaction was carried out as described in example 4 at the paragraph: *Evaluation of the enzymatic activity,* with the following variations: to 100 mg of 2-ethyl-hexanonitrile dissolved in 250 µl of methanol, were added 5 ml of KH₂PO₄ buffer, pH 7.7, containing 2.66% of Triton X-100 and 100 mg of wet bacterial pellet, induced with valeronitrile 0,25% prepared as described in example 3. The solution was incubated at 30°C and agitated. After 32 hours of reaction, the Gas-chromatograph analyses showed the presence of (R)-2-ethylhexane amide (52%) and of (S)-(+)-2-ethylhexanoic acid (45%, e.e.> 98) and with an enzymatic activity of 3.61 IU g⁻¹ of wet cells.

### Example 10. Conversion of aromatic nitriles utilising the bacterial pellet of strain NCIMB 41108, induced with valeronitrile, as a biocatalyst.

### 10.1. Production of benzoic acid starting from benzonitrile.

The reaction was carried out according to the reaction scheme of example 4, with the following variations: to 50 mg of benzonitrile dissolved in 250 µl of methanol, were added 5 ml of KH₂PO₄ buffer, pH 7.7, containing 2.66% of Triton X-100 and 250 mg of wet bacterial pellet, induced with valeronitrile 0,25%, prepared as described in example 3. The solution was incubated at 30°C with agitation. After 17 hours of reaction the HPLC analysis showed the presence of benzonitrile (40%) and of benzoic acid (60%), with an enzymatic activity of *1.14 IU g*^{*-1*} of wet cells.

### 10.2. Production of benzoic acid starting from benzamide.

The reaction was carried out as described in example 4 in the paragraph: *Evaluation of the enzyme activity,* with the following variations: to 50 mg of benzamide dissolved in 250 µl of methanol, were added 5 ml of KH₂PO₄buffer, pH 7.7, containing 2.66% of Triton X-100 and 250 mg of wet bacterial pellet, induced with valeronitrile 0,25%, prepared as described in example 3. The solution was incubated at 40°C and agitated. After 17 hours, the first HPLC analyses were performed showing that the conversion of amide into benzoic acid was already quantitative.

## Claims

1. A micro-organism belonging to the genus *Agrobacterium radiobacter,* capable of converting nitriles and/or amides into their respective acids.

2. The micro-organism according to claim 1, mutagenised and **characterised by** being positive for the enzymatic marker catalase and negative for the enzymatic marker oxidase.

3. The mutagenised micro-organism *Agrobacterium radiobacter* 30"60 according to claims 1 or 2 deposited with NCIMB with accession number 41108.

4. Process for the conversion of nitriles into their respective acids **characterised by** the fact of using the micro-organisms according to the claims 1-3.

5. The process according to claim 4 wherein this conversion is enantioselective.

6. The process according to claims 4 or 5 wherein this conversion comprises the phase a) of conversion of nitriles to amides and that of b) of conversion of amides to acids.

7. Process for the conversion of amides into their respective acids **characterised by** the fact of using the micro-organisms according to claims 1-3.

8. The process according to claim 7 wherein the conversion is enantioselective.

9. The process according to claim 7 or 8 **characterised by** the fact that these amides are selected from aliphatic amides, arylalkyl amides and aromatic amides.

10. The process according to the claims 4-6 **characterised by** the fact that these nitriles are selected from: arylalkyl nitriles, aromatic nitriles and aliphatic nitriles.

11. The process according to claims 9 or 10 wherein these arylalkyl nitriles or these arylalkyl amides are α-methyl-arylacetonitriles ("arylpropionitriles") or α-methylaryl acetamides ("arylpropionamides"), preferably precursors of profens.

12. The process according to claim 11 for the enantioselective preparation of S-(+)-2-(3-benzoylphenyl)propionic acid (pharmacologically active enantiomer of ketoprofen), of S-α-methyl-3-phenoxybenzen-acetic acid (pharmacologically active enantiomer of fenoprofen), of S-2-fluoro-α-methyl[1,1'-diphenyl]-4-acetic acid (pharmacologically active enantiomer of flurbiprofen), of S-4-(1,3)-dihydro-1-oxo-2H-isoindol-2yl)-α-methylbenzene acetic acid (pharmacologically active enantiomer of indoprofen), of S-α-methyl-4-(2-methylpropyl)benzene acetic acid (pharmacologically active enantiomer of ibuprofen) or of S-α-methyl-4-(2-thienylcarbonyl)benzene acetic acid (pharmacologically active enantiomer of suprofen), in which the racemic form of the respective α-arylpropionitrile or α-arylpropionamide precursor is converted into the respective S-acid and with the proviso that, for the production of S-suprofen, the micro organisms are neither induced with isobutyronitrile nor with ε-caprolactam.

13. The process according to claim 11 for the enantioselective preparation of R-α-methyl-4-(2-thienylcarbonyl)benzene acetic acid (R-suprofen).

14. The process according to claims 9 or 10 where these aliphatic nitriles or aliphatic amides are precursors of 2-methylbutyrric acid, of 2-methylpentanoic acid, of 3-methyl-pentanoic acid and of 2-metylhexanoic acid.

15. The process according to claims 9 or 10 **characterised by** the fact that these arylalkyl nitriles and these arylalkyl amides are selected from: mandelonitrile or mandelamide as precursors of mandelic acid, β-(aminomethyl)-4-chlorobenzene-propionitrile or β-(aminomethyl)-4-chlorobenzene-propionamide as precursors of baclofen and α-phenyl-2-piperidylacetonitrile or α-phenyl-2-piperidylacetamide or α-phenyl-2-piridylacetonitrile or α-phenyl-2-piridylacetamide as precursors of methylphenidate.

16. The process according to claims 9 or 10 **characterised by** the fact that these aromatic nitriles and these aromatic amides are selected from: benzonitrile or benzamide, 2-furylnitrile or 2-furylamide, 2-piridylnitrile or 2-piridylamide.

17. The process for the conversion of nitriles to amides **characterised by** the fact of using the micro-organisms according to claims 1-3.

18. The process according to claim 17 **characterised by** the fact that these nitriles are selected from: arylalkyl nitriles, aromatic nitriles or aliphatic nitriles.

19. The process according to claim 18 in which the conversion is enantioselective.

20. The process according to claim 19 wherein these arylalkyl nitriles are α-methyl-arylacetonitriles ("arylpropionitriles"), preferably precursors of profen-amides.

21. The process according to claim 19 for the enantioselective preparation of S-(+)-2-(3-benzoylphenyl)propionic amide, of S-α-methyl-3-phenoxybenzene-acetamide, of S-2-fluoro-α-methyl[1,1'-diphenyl]-4-acetamide, of S-4-(1,3)-dihydro-1-oxo-2H-isoindol-2yl)-α-methylbenzene acetamide, of S-α-methyl-4-(2-methylpropyl)benzene acetamide or of S-α-methyl-4-(2-thienylcarbonyl)benzene acetamide, in which the racemic form of the respective α-arylpropionitrile precursor is converted into the respective S-amide and with the proviso that, for the production of S-α-methyl-4-(2-thienylcarbonyl)benzene acetamide, the micro organisms are neither induced with isobutyronitrile nor with ε-caprolactam.

22. The process according to claim 18 where these aliphatic nitriles are precursors of 2-methylbutyrramide, of 2-methylpentanamide, of 3-methyl-pentanamide and of 2-metylhexanamide.

23. The process according to claim 18 **characterised by** the fact that these arylalkyl nitriles are selected from: mandelonitrile, β-(aminomethyl)-4-chlorobenzene-propionitrile, α-phenyl-2-piperidylacetonitrile or α-phenyl-2-piridylacetonitrile.

24. The process according to claim 18 **characterised by** the fact that these aromatic nitriles are selected from: benzonitrile, 2-furylnitrile or 2-piridylnitrile.

25. Process for the regioselective hydratation and/or hydrolysis of dinitriles,
**characterised by** the fact of using the micro-organisms according to claims 1-3.

26. The process according to claim 25 for the regioselective conversion and preferably enantioselective conversion of dinitriles to give the respective mononitrile-monoamide and/or mononitrile-monoacid and/or monoamide-monoacid derivatives.

27. The process according to claim 26 where these dinitriles are selected from 2-methylphenylmalononitrile and 1,3-benzodinitrile.

28. The process according to the claims 4-27 **characterised by** the fact that these micro-organisms are induced by preferably simple nitriles or amides.

29. The process according to claim 28 **characterised by** the fact that these nitrile or amide inducers are selected from the group constituted by: ε-caprolactam, isobutyramide, benzamide, valeramide, butyramide, lactamide, valeronitrile, isovaleronitrile, butyronitrile and isobutyronitrile.

30. The process according to the claims 4-29 **characterised by** the fact that the micro-organisms are used as pure bacterial cultures or as a homogenate or bacterial lysate or as a crude or semi-purified cellular extract.

31. The process according to the claims 4-30 **characterised by** the fact that this conversion is carried out at a pH comprised of between 5 and 9.5, preferably comprised of between 7 and 8.

32. The process according to claim 31 **characterised by** the fact that this conversion is carried out at a temperature comprised of between 5°C and 45°C.

33. The process according to claim 32 where this temperature is comprised of between 28°C and 42°C.

34. The process according to claim 33 where this temperature is comprised of between 28°C-31°C.

35. The process according to claim 33 where this temperature is comprised of between 38°C-42°C.
